# EUROPEAN PATENT SPECIFICATION

(11) **EP 2 514 441 B1**
(45) Date of publication and mention of the grant of the patent: **24.10.2018**
(21) Application number: 10836948.9
(22) Date of filing: 19.05.2010
(51) Int. Cl.: A61K 31/704, A61K 47/61, A61P 35/00

(54) **PASSIVE SOLID TUMOR TARGETING ANTICANCER PRODRUG AND PREPARATION METHOD THEREOF**
PASSIVES AUF FESTE TUMORE GEZIELTES ANTIKREBS-PRODRUG UND HERSTELLUNGSVERFAHREN DAFÜR
PRO-MÉDICAMENT ANTICANCÉREUX PASSIF CIBLANT LES TUMEURS SOLIDES ET SON PROCÉDÉ DE PRÉPARATION

(30) Priority: 18.12.2009 CN 200910311854
(43) Date of publication of application: 24.10.2012
(73) Proprietor: Chongqing Lummy Pharmaceutical Co., Ltd., Chongqing 401123 (CN)
(72) Inventor: TANG, Xiaohai, Chongqing 401123 (CN); QIU, Yu, Chongqing 401123 (CN); SONG, Xin, Chongqing 401123 (CN)
(74) Representative: Huang, Liwei
(86) International application number: PCT/CN2010/072908
(87) International publication number: WO 2011/072510

(56) References cited:
- WO-A1-2010/042823
- CN-A- 101 045 163
- CN-A- 101 134 109
- CN-A- 101 134 109
- CN-A- 101 433 723
- CHENG M ET AL: "Preparation and Lymphatic Targeting Research of Targeting Antitumor Drug : Pectin-Adriamycin Conjugates", SHENGWU YIXUE GONGCHENGXUE ZAZHI = JOURNAL OF BIOMEDICAL ENGINEERING, CHENGDU, CN, vol. 26, no. 3, 1 June 2009 (2009-06-01), pages 569-574, XP008148136, ISSN: 1001-5515
- TANG XIAO-HAI ET AL: "Synthesis, characterization, and in vitro and in vivo evaluation of a novel pectin-adriamycin conju", BIOORGANIC & MEDICINAL CHEMISTRY, vol. 18, no. 4, 7 January 2010 (2010-01-07), pages 1599-1609, XP028649829, ISSN: 0968-0896, DOI: 10.1016/J.BMC.2009.12.076
- CHENG MING ET AL.: 'Preparation and Lymphatic Targeting Research of Targeting Antitumor Drug : Pectin-Adriamycin Conjugates' JOURNAL OF BIOMEDICAL ENGINEERING vol. 26, no. 3, June 2009, pages 569 - 574, XP008148136

## Description

### Field of the Invention

The invention relates to a passive solid tumor-targeted anticancer prodrug and a preparation method thereof, belonging to the field of antitumor drugs.

### Description of the Related Art

Microvascular endothelia of normal tissues have compact gap and complete structure, thus macromolecules and lipid particles are difficult to pass through vascular wall. Compared with capillaries of normal tissues, solid tumors are characterized by euangiotic tissues, irregular shape and structure, dilation and microvascular wall deficiency of capillaries, loose arrangement of endothelial cells, poor structural integrity, wide junction gap between endothelial cells and lymphatic return deficiency, causing macromolecular substances and lipid particles to have selective enhanced permeability and retention, the phenomenon is called enhanced permeability and retention effect of solid tumor tissues (hereinafter referred to as EPR effect). Scanning electronic microscope shows that microvascular endothelial cell junction gap in human colonic adenocarcinoma is up to 400nm, while the average microvascular endothelial cell junction gap in normal tissues is less than 100nm. Pathological structural characteristics of solid tumor tissues cause macromolecular anticancer drugs to be passively targeted to or select solid tumors, and be largely distributed in tumor tissues after systemic administration, which is also known as passive solid tumor-targeted property. However, small molecular anticancer drugs can freely pass through vascular walls of normal tissues and tumor tissues, and have consistent drug distribution in normal tissues and tumor tissues, which are one of important reasons for poor anticancer effect selectivity and stronger toxic and side effects. Therefore, small molecular anticancer do not have passive targeting effect. Greish K, etc. reported that macromolecular substances with relative molecular weight more than 40,000 can overcome renal filtration and clearance and have longer plasma half-life, the extension of systemic circulation time of macromolecular substances benefits the realization of EPR effect and reduce administration frequency as well.

Pectin is generally a natural macromolecular polysaccharide polymer, widely distributed in plant cell walls, and an acidic macromolecular polysaccharide consisting of α-(1→4)-D-pyranylgalacturonic unit (Hyunjo Kim, et al. International Journal of Pharmaceutics, 1998, 161:149-159). Pectin can improve the immune function of hosts by enhancing mononuclear phagocyte system, activating macrophages, T cells, B cells, NK cells and complement system, promoting cytokine secretion, enhancing immunity of erythrocytes, etc.; and exert direct anticancer effects by changing the growth characteristics of solid cancer cell membrane, affecting signal transmission path in solid cancer cells and anti-free radical effect, inducing differentiation and apoptosis, inhibiting synthesis of nucleic acid and protein of the solid cancer cells, affecting ultrastructure of the solid cancer cells, affecting oncogenes and antimutation effect, and inhibiting vascularization of solid cancer (Chinese Journal of Information on Traditional Chinese Medicine, 1999, 5:64). The inventor began the research of a macromolecular anticancer prodrug using pectin as a carrier since 2005, and applied for 3 Chinese invention patents with application numbered 200610020596.7, 200710201724.2 and 200810306465.3 respectively. The anticancer drug using small molecular pectin can freely pass through vascular walls of normal tissues and tumor tissues and be excreted easily. However, as the anticancer drug has consistent drug distribution in normal tissues and tumor tissues and poor anticancer effect selectivity, and stays in human body for a short time, the administration frequency required is higher. The anticancer prodrug prepared by using macromolecular pectin as a carrier can produce EPR-based passive tumor-targeted property, has remarkable cumulative effect in tumor tissues, and multiple administration does not affect the distribution thereof. As pectin is difficult to be degraded in body and can only be excreted through the kidney, excessive macromolecular pectin accumulating in body is likely to be deposited in the lung, kidney and other tissues and organs, affecting functions thereof, and specific consequence has not been reported.

CN101134109A (i.e. application numbered 200710201724.2) discloses anticancer prodrugs formed by reacting small molecular pectin with anticarcinogen, whereby the pectin is linked with the anticarcinogen by amide/ester bond. The conjugate formed by small molecular pectin and doxorubicin could target lymphatic, liver or lung.

CHENG M ET AL (Preparation and Lymphatic Targeting Research of Targeting Antitumor Drug: Pectin-Adriamycin Conjugates" SHENGWU YIXUE GONGCHENGXUE ZAZHI = JOURNAL OF BIOMEDICAL ENGINEERING, vol.26, no.3, pages 569-574) discloses conjugates formed by pectin and doxorubicin, which are linked by amide bond. The conjugate could release the doxorubicin in lymph nodes with lymphatic targeting characteristics.

### Summary of the Invention

The technical problem to be solved by the inventions is EPR effect-based passive tumor-targeted property of a pectin anticancer prodrug, and to release the drug and decompose the carrier into small molecules after entering solid tumor tissues, benefiting excretion.

In order to realize the purpose of the invention, the technical solution of the invention is realized by:
cutting macromolecular pectin into small molecular pectin with Mw of 5,000 - 45,000 (preferably 10,000 - 30,000), reacting small molecular pectin with MW of 5,000 - 45,000 with doxorubicin to obtain a pectin-doxorubicin conjugate with Mw of 100,000 - 1,000,000 (preferably 200,000 - 800,000, further preferably 400,000 - 600,000), preparing the pectin-doxorubicin conjugate into a suspension (adding the pectin-doxorubicin conjugate to water, adding PVP and glycerol and evenly mixing), and treating the suspension in an ultra-high pressure nano homogenizer to obtain the passive solid tumor-targeted anticancer prodrug with particle size of 100nm - 200nm (preferably 130nm - 180nm) and melting point of 220 - 245°C, wherein in the pectin-doxorubicin conjugate, the pectin and doxorubicin are linked by an amide bond, and the pectin is linked by an ester bond formed by condensing carboxyl groups and hydroxyl groups of pectin molecules. After entering solid tumor tissues, the anticancer prodrug can produce EPR-based passive tumor-targeted property, have remarkable cumulative effect in the tumor tissues, longer plasma half-life and extended systemic circulation time, release the drug and decompose the carrier into small molecules, benefiting excretion. Molecular weight of the invention is weight average molecular weight (Mw).

Water solubility of the passive solid tumor-targeted anticancer prodrug is 42mg/L.

A method for preparing the passive solid tumor-targeted anticancer prodrug comprises the following steps:
a. dissolving small molecular pectin with Mw of 5,000 - 45,000 (preferably 10,000 - 30,000) in water, adding doxorubicin hydrochloride, reacting with EDC·HCl after mixing evenly, dialyzing and drying to obtain a pectin-doxorubicin conjugate with Mw of 100,000 - 1,000,000;
b. adding the pectin-doxorubicin conjugate to water, adding PVP and glycerol (or adding a certain amount of lecithin or DMSO, with the amount added not more than 2% of the pectin-doxorubicin conjugate), evenly mixing to prepare a suspension and treating the suspension in an ultra-high pressure nano homogenizer to obtain the passive solid tumor-targeted anticancer prodrug with particle size of 100nm - 200nm.

The pectin-doxorubicin conjugate is obtained by reacting the small molecular pectin with the doxorubicin in the presence of the EDC·HCl at 40 - 60°C with pH of 5 - 7.

Furthermore, in step b, the dosage of the PVP is 1 - 6 times of the mass of the pectin-doxorubicin conjugate, and the dosage of the glycerol is 0.1 - 0.8% of the mass of the pectin-doxorubicin conjugate.

A preferable treatment method is to treat the suspension in the ultra-high pressure nano homogenizer for 3 times at 120mpa for the first time, 180mpa for the second time and 190mpa for the third time.

The small molecular pectin is obtained by dissolving pectin in water, reacting the pectin with NaOH solution with pH of 13, adjusting the pH to neutral with concentrated hydrochloric acid and cutting off molecular weight.

The passive solid tumor-targeted anticancer prodrug of the invention is hydrolyzed in lyase, breaking the amide bond and releasing doxorubicin. A filter cake cut off after hydrolysis and ultrafiltration is repeatedly washed with 95% ethanol until the liquid is not red so as to remove residual doxorubicin, the solvent is evaporated to dryness, distilled water is added to dissolve precipitate, and the molecular weight determined by gel permeation chromatography is 10,000 - 30,000.

The cell inhibition rate of the passive solid tumor-targeted anticancer prodrug (pectin-doxorubicin conjugate) injection of the invention for humanized lung cancer cells NCI-H446 and A549 is 65.23% and 68.52% respectively, which is equivalent to that of doxorubicin hydrochloride (63.33% and 67.62%) at the same doxorubicin concentration (2mg/ml). In the efficacy study of melanoma B16 pulmonary metastasis model mice, the life span of tumor-bearing mice in the pectin-doxorubicin conjugate group is 42.3±12.4 days, which is remarkably higher than that of the doxorubicin hydrochloride group (23.1±10.2 days).

### Brief Description of the Drawings

Figure 1 is an ultraviolet spectrogram;
Figure 2 is an infrared spectrogram; a represents doxorubicin, b represents pectin-doxorubicin conjugate, c represents pectin, and d represents physical mixture of pectin and doxorubicin;
Figure 3 is particle size distribution of the passive solid tumor-targeted anticancer prodrug of the invention;
Figure 4 is lysosome hydrolysis test of the passive solid tumor-targeted anticancer prodrug of the invention; series 1 is an experimental group and series 2 is a blank group, the horizontal coordinate is time (h) and the vertical coordinate is drug release percentage (%); and
Figure 5 is the effect of the passive solid tumor-targeted anticancer prodrug of the invention on life span of lung cancer-bearing mice.

### Detailed Description of the Preferred Embodiments

A method for preparing a passive solid tumor-targeted anticancer prodrug comprises the following steps: cutting macromolecular pectin into small molecular pectin with Mw of 5,000 - 45,000 (preferably 10,000 - 30,000), then synthesizing small molecular pectin-doxorubicin (through amide bond linkage), and condensing carboxyl groups and hydroxyl groups of pectin molecules to form macromolecules, and treating the macromolecules in an ultra-high pressure nano homogenizer to obtain the passive solid tumor-targeted anticancer prodrug with particle size of 100nm - 200nm and weight molecular weight of 100,000 - 1,000,000.

### Specific preparation method:

### 1. Preparation of small molecular pectin: dissolving pectin in distilled water, reacting pectin with NaOH, adjusting pH to neutral with concentrated hydrochloric acid, cutting off to obtain small molecular pectin with molecular weight (Mw) of 5,000 - 45,000 (preferably 10,000 - 30,000).

One example: weighing 13.3g pectin, adding 1L distilled water, mixing to dissolve, adjusting pH to 13 with 5M NaOH, reacting at 65°C for 10h, and stopping the reaction; adjusting reaction solution to neutral with hydrochloric acid, filtering by a millipore with cut-off molecular weight of 30,000, filtering the filtrate by a millipore with cut-off molecular weight of 10,000, collecting impermeable solid with cut-off molecular weight of 10,000, reducing pressure and concentrating to dryness, and drying under vacuum to obtain small molecular pectin with molecular weight of 10,000 - 30,000.

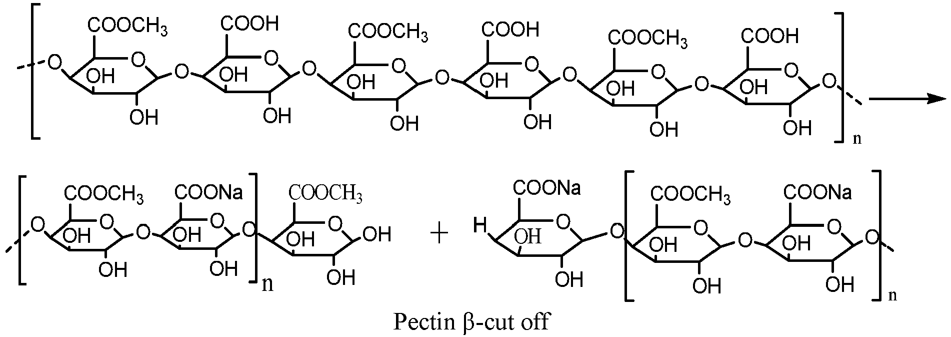

### 2. Loading of doxorubicin and crosslinking of small molecular pectin:

### 2.1 Reaction formula

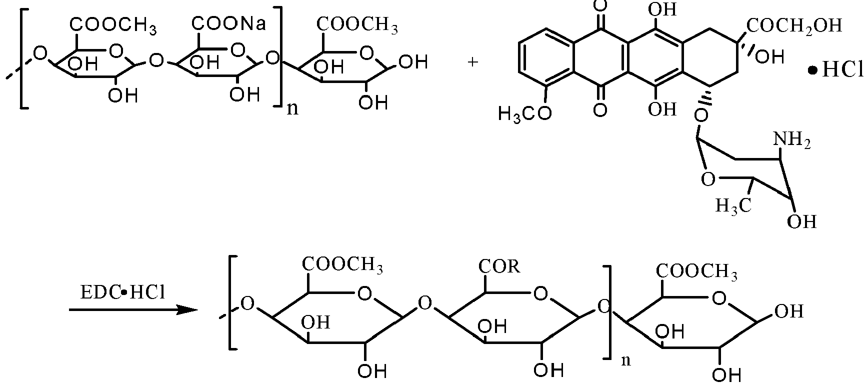

Where

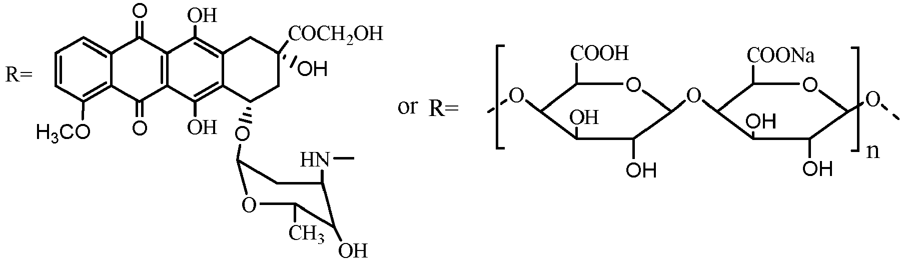

### 2.2 Experimental procedures

The experimental procedures were as follows: dissolving small molecular pectin with molecular weight of 10,000 - 30,000, reacting with doxorubicin hydrochloride solution in the presence of 1-ethyl-3-(3-dimethylaminopropyl)carbodiimide hydrochloride (EDC·HCl) at 40 - 60°C with pH of 5 - 7, dialyzing and drying to obtain a red brown water-insoluble solid (pectin-doxorubicin conjugate) with solubility of 42mg/L and melting point of 220-245°C.

The drug loading rate measured was 15 - 35%, and the molecular weight (Mw) was 100,000 - 1,000,000, preferably 200,000 - 800,000, and further preferably 400,000 - 600,000.

Back analysis of ultraviolet-visible spectrophotometry showed that the pectin was not absorbed, doxorubicin had the maximum absorption peak at 479.5nm, mixture of the pectin and the doxorubicin had the maximum absorption peak at 488.5nm, the pectin-doxorubicin conjugate P(A)ₙ had the maximum absorption peak at 498nm, and absorption red shift occurred, indicating chemical bond coupling between doxorubicin and the pectin.

Scanning on infrared spectrum, compared with the pectin, the P(A)ₙ had hybrid absorption peak of amide band I and amide band II at 1620cm⁻¹, and compared with ester bond peak at 1750cm⁻¹, the peak area ratio increased remarkably, indicating that the pectin was cross-linked by an ester bond, and remarkable anthracycline characteristic absorption peak of doxorubicin appeared at 1100cm⁻¹ and 1017cm⁻¹, and absorption peak of primary amide appeared at 1411.23, indicating that the pectin and the doxorubicin were linked by an amide bond.

The red brown solid was subject to grinding treatment, added with a certain amount of PVP, and treated in an ultra-high pressure nano homogenizer (type T-200D manufactured by Langfang General Machinery Manufacturing Co., Ltd. in Hebei) to obtain a passive solid tumor-targeted anticancer prodrug with molecular weight of 100,000 -1,000,000 and particle size of 100nm - 200nm.

### 3. The insoluble macromolecular pectin-doxorubicin conjugate was subject to grinding treatment, added with a certain amount of PVP and treated in the ultra-high pressure nano homogenizer to obtain the passive solid tumor-targeted anticancer prodrug with molecular weight of 100,000 -1,000,000 and particle size of 100nm - 200nm.

The examples below are for further illustration of the invention.

### Example 1 Preparation of the passive solid tumor-targeted anticancer drug P(A)ₙ of the invention

### 1. Preparation of small molecular pectin:

The preparation comprised the following steps: weighing 13.3g pectin, adding 1L distilled water, mixing to dissolve, adjusting pH to 13 with 5M NaOH, reacting at 65°C for 10h, and stopping the reaction; adjusting reaction solution to neutral with concentrated hydrochloric acid, filtering by a millipore with cut-off molecular weight of 30,000, filtering the filtrate by a millipore with cut-off molecular weight of 10,000, collecting impermeable solid with cut-off molecular weight of 10,000, reducing pressure and concentrating to dryness, and drying under vacuum to obtain small molecular pectin with molecular weight of 10,000 - 30,000.

### 2. Loading of doxorubicin and crosslinking of small molecular pectin:

The loading and crosslinking comprised the following steps: weighing and adding 1g small molecular pectin-doxorubicin with molecular weight of 10,000 - 30,000 to a reaction flask, adding 100ml water, mixing to dissolve, weighing 0.5g doxorubicin hydrochloride, adding 50ml distilled water for ultrasonic dissolution, adding the doxorubicin hydrochloride solution to the reaction flask, and washing doxorubicin adhered to the reaction flask with further 50ml distilled water; weighing and adding 1g EDC·HCl to the reaction flask, rising the temperature to 50°C, reacting for 6.5h, loading in a dialysis bag with cut-off molecular weight (Mw) of 3500 for dialysis for 1d after reaction, and changing the distilled water once every 3h; evaporating the solvent to dryness, and drying under vacuum for 12h to obtain a red brown solid, i.e. 1.1g water-insoluble macromolecular insoluble pectin-doxorubicin conjugate with solubility of 42mg/L and melting point of 220°C - 245°C.

### Determination of drug loading rate by spectrophotometry:

Establishment of a standard curve: accurately preparing standard doxorubicin hydrochloride solution at concentrations of 10.00, 20.00, 30.00, 40.00 and 50.00µg/mL respectively, and determining absorbance at 479.5 nm (determined by spectral scanning using an ultraviolet-visible spectrophotometer).

Determination of drug loading rate of samples: accurately weighing a certain amount of P(A)ₙ, dissolving the P(A)ₙ in secondary water to prepare the solution to be measured, determining the absorbance, and measuring the drug loading rate to be 21.4%.

### Structural characterization

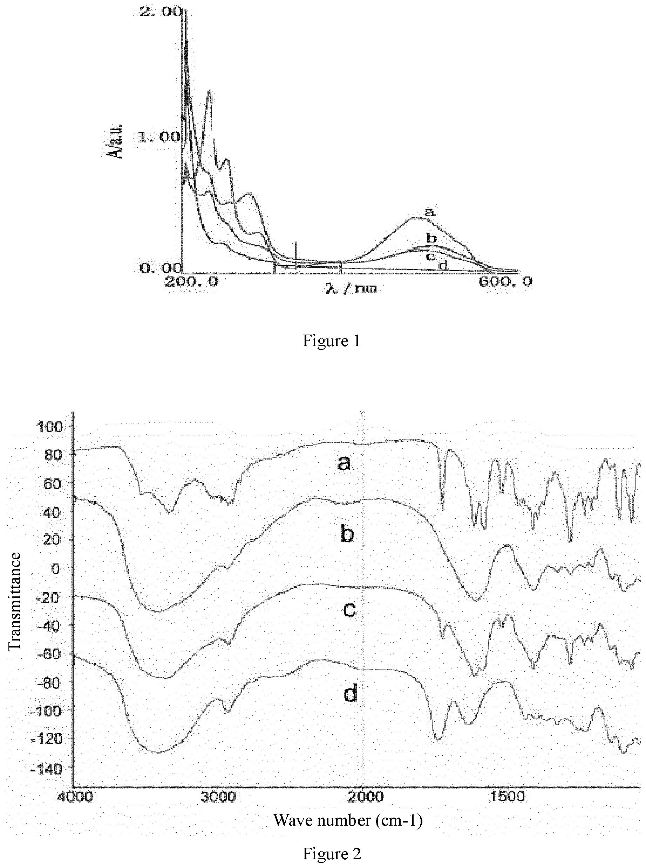

In order to avoid the effect of carboxylate in the P(A)ₙ on the ester bond and the amide bond, the P(A)ₙ was subject to non-salt treatment to prepare a prodrug sample. The doxorubicin, the pectin, sample of the invention and mixture of the doxorubicin and the pectin were dissolved in the secondary water, and ultraviolet spectrum and infrared spectrum are as shown in Figure 1 and Figure 2 respectively.

In Figure 1, doxorubicin has the maximum absorption peak at 479.5nm, mixture of the pectin and the doxorubicin has the maximum absorption peak at 488.5nm, the P(A)ₙ has the maximum absorption peak at 498nm, and the pectin is not absorbed. Absorption red shift occurs to the P(A)ₙ at 498nm, indicating that chemical bond coupling between the doxorubicin and the pectin.

In Figure 2, compared with the pectin, the pectin-doxorubicin has hybrid absorption peak of amide band I and amide band II at 1620cm⁻¹, and compared with ester bond peak at 1750cm⁻¹, the peak area ratio increases remarkably, indicating that the pectin is cross-linked by ester bond and remarkable anthracycline characteristic absorption peak of the doxorubicin appears at 1100cm⁻¹ and 1017cm⁻¹, and absorption peak of primary amide appears at 1411.23, indicating that the pectin and the doxorubicin are linked by an amide bond.

### 3. Preparation of the passive solid tumor-targeted anticancer prodrug:

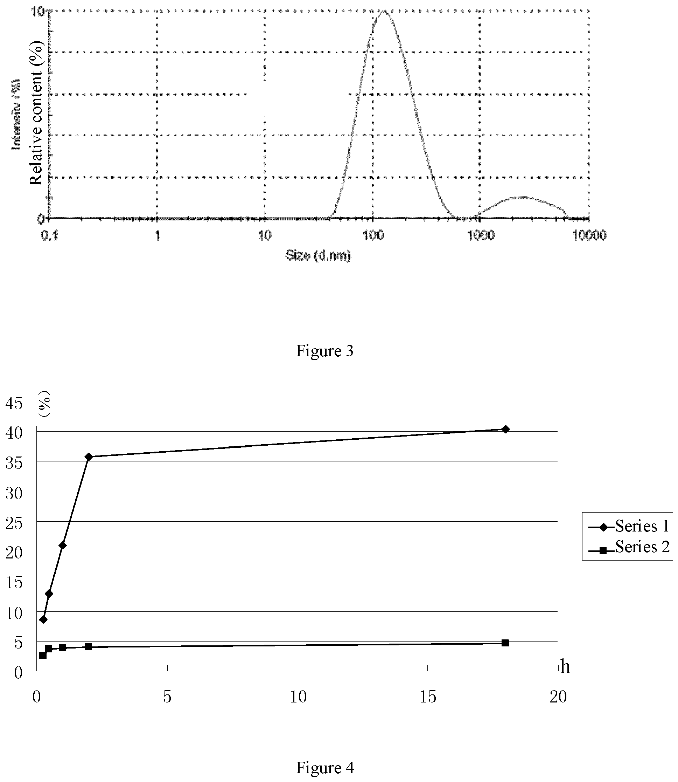

0.468g macromolecular insoluble pectin-doxorubicin conjugate (21.4% of drug loading rate) was added with 1g PVP, 3ml glycerol and 50ml water, subject to grinding treatment to prepare a suspension, and treated in an ultra-high pressure nano homogenizer (type T-200D manufactured by Langfang General Machinery Manufacturing Co., Ltd. in Hebei). The suspension was treated in the ultra-high pressure nano homogenizer for 3 times at 120mpa for the first time, 180mpa for the second time and 190mpa for the third time. Particle size distribution after treatment in the ultra-high pressure nano homogenizer is as shown in Figure 3.

The suspension was treated in the ultra-high pressure nano homogenizer to obtain the passive solid tumor-targeted anticancer prodrug with molecular weight of 100,000 -1,000,000 and particle size of 100nm - 200nm.

### Example 2 Preparation of the passive solid tumor-targeted anticancer drug P(A)ₙ of the invention

### 1. Preparation of small molecular pectin:

The preparation comprised the following steps: weighing 13.3g pectin, adding 1L distilled water, mixing to dissolve, adjusting pH to 13 with 5M NaOH, reacting at 65°C for 10h, and stopping the reaction, adjusting reaction solution to neutral with concentrated hydrochloric acid, filtering by a millipore with cut-off molecular weight of 10,000, dialyzing the filtrate with a dialysis bag with cut-off molecular weight of 7,000, collecting dislysate, reducing pressure and concentrating to dryness, and drying under vacuum to obtain small molecular pectin with molecular weight of 7,000 - 10,000.

### 2. Loading of doxorubicin and crosslinking of small molecular pectin:

The loading and crosslinking comprised the following steps: weighing and adding 1g small molecular pectin-doxorubicin with molecular weight of 7,000 - 10,000 to a reaction flask, adding 100ml water, mixing to dissolve, weighing 0.5g doxorubicin hydrochloride, adding 50ml distilled water for ultrasonic dissolution, adding the doxorubicin hydrochloride solution to the reaction flask, and washing doxorubicin adhered to the reaction flask with further 50ml distilled water; weighing and adding 1g EDC·HCl to the reaction flask, rising the temperature to 50°C, reacting for 6.5h, loading in a dialysis bag with cut-off molecular weight (Mw) of 3500 for dialysis for 1d after reaction, and changing the distilled water once every 3h; evaporating the solvent to dryness, drying under vacuum for 12h to obtain a red brown solid, i.e. 1.2g macromolecular insoluble pectin-doxorubicin conjugate with the drug loading rate of 24.2%.

0.468g macromolecular insoluble pectin-doxorubicin conjugate was added with 1g PVP, 3ml glycerol and 50ml 2% lecithin solution as solvents, subject to grinding treatment to prepare a suspension, and treated in an ultra-high pressure nano homogenizer (type T-200D manufactured by Langfang General Machinery Manufacturing Co., Ltd. in Hebei). The suspension was treated in the ultra-high pressure nano homogenizer for 3 times at 120mpa for the first time, 180mpa for the second time and 190mpa for the third time.

The passive solid tumor-targeted anticancer prodrug with molecular weight of 100,000-1,000,000 and particle size of 100nm-200nm was obtained.

### Example 3 Preparation of the passive solid tumor-targeted anticancer drug P(A)ₙ of the invention

### 1. Preparation of small molecular pectin:

The preparation comprised the following steps: weighing 13.3g pectin, adding 1L distilled water, mixing to dissolve, adjusting pH to 13 with 5M NaOH, reacting at 65°C for 10h, and stopping the reaction; adjusting reaction solution to neutral with concentrated hydrochloric acid, filtering by a millipore with cut-off molecular weight of 50,000, dialyzing the filtrate with a dialysis bag with cut-off molecular weight of 20,000 for 48h, and changing the distilled water once every 3h, reducing pressure and concentrating the dialysate to dryness, and drying under vacuum to obtain small molecular pectin with molecular weight of 20,000 - 50,000.

### 2. Loading of doxorubicin and crosslinking of small molecular pectin:

The loading and crosslinking comprised the following steps: weighing and adding 1g small molecular pectin with molecular weight of 20,000 - 50,000 to a reaction flask, adding 100ml water, mixing to dissolve, weighing 0.5g doxorubicin hydrochloride, adding 50ml distilled water for ultrasonic dissolution, adding the doxorubicin hydrochloride solution to the reaction flask, and washing doxorubicin adhered to the reaction flask with further 50ml distilled water; weighing and adding 1g EDC·HCl to the reaction flask, rising the temperature to 50°C, reacting for 6.5h, loading in a dialysis bag with cut-off molecular weight (Mw) of 3500 for dialysis for 1d after reaction, and changing the distilled water once every 3h; evaporating the solvent to dryness, drying under vacuum for 12h to obtain a red brown solid, i.e. 1.2g macromolecular insoluble pectin-doxorubicin conjugate with the drug loading rate of 25.2%.

0.468g of macromolecular insoluble pectin-doxorubicin conjugate was added with 1g PVP and 50ml mixture of water and DMSO (water:DMSO=0.75:0.25) to prepare a suspension, and the suspension was treated in an ultra-high pressure nano homogenizer (type T-200D manufactured by Langfang General Machinery Manufacturing Co., Ltd. in Hebei). The suspension was treated in the ultra-high pressure nano homogenizer for 3 times at 120mpa for the first time, 180mpa for the second time and 190mpa for the third time.

The passive solid tumor-targeted anticancer prodrug with molecular weight of 100,000-1,000,000 and particle size of 100nm-200nm was obtained.

### Test example 1 Lysosome hydrolysis test of the passive solid tumor-targeted anticancer prodrug of the invention

Lysosome source: purified lysosome was extracted by referring to *Experimental Method and Technique for Cell Biology.*

Experimental group: a 25mL conical flask was added with 10mL phosphate buffer solution (PBS) (pH=5), 10mg 1mg/mL passive solid tumor-targeted anticancer prodrug prepared in Example 2 and 0.4mL sucrose (0.25mol/L) suspension of lysosome, and placed in a 37°Cthermostat for incubation in a dark place on a shaker.

Lysosome was not added to the control group, but other conditions were the same.

A 0.5mL sample was taken at heat insulation for 0.25h, 0.5h, 1h, 2.5h and 18h respectively, and then 0.5mL ultrapure water, 0.2mL 1mol/L Na₂CO₃NaHCO₃ (pH 9.8) buffer solution and 2.5mL CHCl₃-MeOH (3:1) were successively added after sampling, evenly mixed and centrifuged at 3,500rpm for 20min, then doxorubicin was distributed at an organic phase.

The content of doxorubicin was determined by high performance liquid chromatography. Chromatographic column: Phenomenex Luna C₁₈(250×4.6mm, 5µm); mobile phase: methanol:acetonitrile:phosphate buffer salt=7:4:6; detection wavelength: 480nm; flow rate: 0.8mL/min.

Experimental results are as shown in Figure 4: the maximum release of the experimental group was 35%, the release reached 30% after 6h, was basically stable at 30% within 6 - 30h, the maximum release of the control group was 7%, the initial release and final release in the whole release process were low, indicating that he passive solid tumor-targeted anticancer prodrug of the invention hydrolyzed in lyase, and the amide bond broke to release the doxorubicin.

Determination of the molecular weight of small molecular pectin after lipid reduction by alkali method and ultrafiltration:
A filter cake held back after ultrafiltration was collected, repeatedly washed with 95% ethanol until the liquid was not red, the solvent was evaporated to dryness, distilled water was added to dissolve precipitate, and the molecular weight was determined by gel permeation chromatography.

Chromatographic conditions: Aglient1100 series HPLC, 1362A parallax detector and G1310A unit pump; chromatographic column: Ultrahydrogel™ linear column (7.8x300mm, Waters); column temperature: 40°C; temperature of flow cell: 35°C; mobile phase: 0.005M KNO₃; flow rate: 0.5mL/min; sample concentration: 5mg/mL, 0.05% sodium azide is used for dissolution; and sample size: 20µL.

Preparation of a standard curve: glucan was used for acting on standard substances Dextrans (Mw = 5,000, 25,000, 50,000, 80,000, 270,000).

The molecular weight measured was 10,000 - 30,000.

Determination of the molecular weight of high ester pectin of commercially available orange in the same conditions: absolute molecular weight measured was 30,000 - 600,000.

### Test example 2 Observation of inhibition of the passive solid tumor-targeted anticancer prodrug on growth activity of tumor cell lines in vitro by MTT method

### 1. Test drug

Doxorubicin hydrochloride was purchased from Zhejiang Haizheng Pharmaceutical Co., Ltd., and prepared to contain 2mg/ml doxorubicin by dissolving in physiological saline.

The passive solid tumor-targeted anticancer prodrug used in the test was prepared in the test example 1 (equivalent to 2mg/ml doxorubicin).

2. Method: cell solution (with concentration of 50,000 cells/ml) growing in logarithmic growth phase cultured with RPMI 1640 culture medium was inoculated on a 96 well plate with 0.1ml/well, and subject to corresponding drug treatment after incubation for 24 hours. After drug treatment for 24 hours, 0.02ml 5mg/ml 3-(4,5-dimethylthiazolyl-2)-2,5-diphenyltetrazolium bromide (MTT) was added for treatment for 4 hours, then culture solution was removed, 0.15ml dimethylsulfoxide (DMSO) was added, and light absorption value was determined at 570nm by a micropplate reader.

Inhibition rate (%)=(1-absorption value/absorption value of blank control group)×100%, and cell inhibition rate of different drugs of each group is as shown in Figure 1.

**Table 1 Inhibition rates of different drugs for various tumor cells**

| Cell line | Inhibition rate (%) | |
|---|---|---|
| | Example 2 | DOX·HCl |
| NCI-H446 | 65.26 | 63.33 |
| A549 | 68.52 | 67.62 |
| 2780-CP | 47.45 | 49.3 |
| 2780-S | 67.14 | 52.8 |
| B16 | 57.01 | 85.9 |

### Test example 3 Experimental research of the passive solid tumor-targeted anticancer prodrug on pulmonary metastasis tumor

### 1. Main materials

Doxorubicin hydrochloride was purchased from Zhejiang Haizheng Pharmaceutical Co., Ltd.

Pectin-doxorubicin conjugate was the passive solid tumor-targeted anticancer prodrug of the invention and a sample of the test example 2 (equivalent to 2mg/ml doxorubicin).

Cells and animals: inbred line C57BL/6 mice of clean grade were purchased from West China Medical Laboratory Animal Center of Sichuan University, female, 6 - 7 weeks old, with weight of 20±3g; allowed for free drinking and eating during the experiment, illumination for 12 hours every day, and mouse cages (5 mice/cage) were ventilated by a central ventilation system. Mouse melanoma cell line B16 was purchased from Shanghai Institute of Cell Biology, and conserved by the laboratory.2. Method:
Cell culture: cells were routinely cultured in culture solution of RPMI 1640 100mL/L fetal bovine serum and penicillin-streptomycin solution (100U/mL penicillin, 100mg/L streptomycin), placed in a 50mL/L CO₂ incubator at 37°C for culture, cells in logarithmic growth phase were collected after 3, 4 stable subcultures, digested by 2.5g/L trypsin, and collected by resuspension of serum-free culture solution, and cell density was adjusted by a serum-free 1640 medium for subsequent use.

Establishment of mouse pulmonary metastatic tumor model: C57BL/6 mice were randomly divided into several groups, tail skin of the mice was disinfected with 750mL/L (mass concentration) ethanol, B16 melanoma cell solution was used for caudal vein injection in the mice, with the dosage of inoculation of 0.1mL.

The drug was administered 4 - 5 days after tumor cells were inoculated in the mice, each mouse was administered twice every week, with dosage of 40 - 50µl/mouse/time. And the life span of tumor-bearing mice was observed.

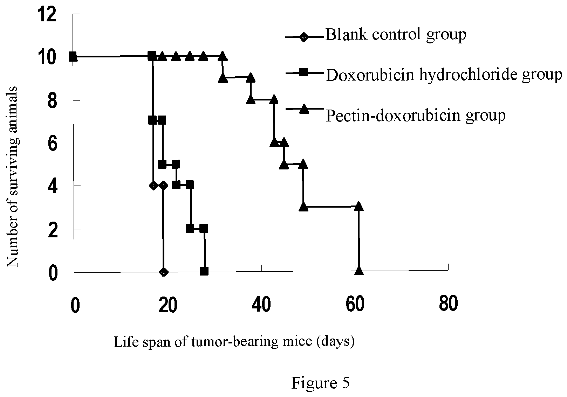

The effect of the passive solid tumor-targeted anticancer prodrug of the invention on life span of lung cancer-bearing mice is as shown in Figure 5. It can be seen from Figure 5 that in the research of efficacy of melanoma B16 pulmonary metastasis model mice, the life span of tumor-bearing mice in the pectin-doxorubicin conjugate group is 42.3±12.4 days, which is remarkably higher than that of the doxorubicin hydrochloride group (23.1±10.2 days).

## Claims

1. A passive solid tumor-targeted anticancer prodrug formed by bonding pectin and doxorubicin, **characterized by** reacting small molecular pectin with Mw of 5,000 - 45,000 with doxorubicin to obtain a pectin-doxorubicin conjugate with Mw of 100,000 - 1,000,000, preparing the conjugate into a suspension, and treating the suspension in an ultra-high pressure nano homogenizer to obtain the passive solid tumor-targeted anticancer prodrug with particle size of 100nm - 200nm and melting point of 220 - 245°C, wherein the pectin and doxorubicin are linked by an amide bond, and the pectin is linked by an ester bond formed by condensing carboxyl groups and hydroxyl groups of pectin molecules.

2. The passive solid tumor-targeted anticancer prodrug of claim 1, **characterized in that** particle size of the passive solid tumor-targeted anticancer prodrug is 130nm - 180nm.

3. The passive solid tumor-targeted anticancer prodrug of claim 1 or 2, **characterized in that** water solubility of the passive solid tumor-targeted anticancer prodrug is 42mg/L.

4. The passive solid tumor-targeted anticancer prodrug of claim 1, **characterized in that** the pectin-doxorubicin conjugate is obtained by reacting the small molecular pectin with doxorubicin in the presence of EDC·HCl at 40 - 60°C with pH of 5 - 7.

5. The passive solid tumor-targeted anticancer prodrug of claim 4, **characterized in that** Mw of the small molecular pectin is 10,000 - 30,000.

6. The passive solid tumor-targeted anticancer prodrug of any of claim 1 to 5, **characterized by** adding the pectin-doxorubicin conjugate to water, adding PVP and glycerol, evenly mixing to prepare the suspension and treating the suspension in the ultra-high pressure nano homogenizer.

7. The passive solid tumor-targeted anticancer prodrug of claim 6, **characterized by** treating the suspension in the ultra-high pressure nano homogenizer for 3 times at 120mpa for the first time, 180mpa for the second time and 190mpa for the third time.

8. The passive solid tumor-targeted anticancer prodrug of claim 7, **characterized in that** dosage of the PVP is 1 - 6 times of the mass of the pectin-doxorubicin conjugate, and dosage of the glycerol is 0.1 - 0.8% of the mass of the pectin-doxorubicin conjugate.

9. A method for preparing a passive solid tumor-targeted anticancer prodrug, **characterized by** comprising the following steps:
a. dissolving small molecular pectin with Mw of 5,000 - 45,000 in water, adding doxorubicin hydrochloride, reacting with EDC·HCl for 3 - 8h after mixing evenly, dialyzing and drying to obtain a pectin-doxorubicin conjugate with Mw of 100,000 - 1,000,000; and
b. adding the pectin-doxorubicin conjugate to water, adding PVP and glycerol, evenly mixing to prepare a suspension and treating the suspension in an ultra-high pressure nano homogenizer to obtain the passive solid tumor-targeted anticancer prodrug with particle size of 100nm - 200nm.

10. The method for preparing the passive solid tumor-targeted anticancer prodrug of claim 9, **characterized in that** the small molecular pectin is obtained by dissolving pectin in water, reacting the pectin with NaOH solution with pH of 13, adjusting pH to neutral with concentrated hydrochloric acid and cutting off molecular weight.

## Patentansprüche

1. Eine passive, stabile, auf einen Tumor abzielende, krebsbekämpfende Prodrug aus gebundenem Pektin und Doximbicin, **gekennzeichnet durch** die Reaktion eines kleinmolekularen Pektins mit einer Molmasse von 5.000 - 45.000 mit Doxombicin, um eine Pektin-Doxorubicin-Kombination mit einer Molmasse von 100.000 - 1.000.000 zu erhalten, Vorbereitung der Kombination zu einer Suspension, sowie Behandlung der Suspension in einem Ultrahochdruck Nano-Homogenisierer, um die passive, stabile, auf den Tumor abzielende, krebsbekämpfende Prodrug mit einer Teilchengröße von 100 nm - 200 nm und einer Schmelztempertaur von 220 - 245°C zu erhalten, wobei das Pektin und Doxombicin durch eine Amidverbindung und das Pektin mit einer Esterverbindung verknüpft ist, die durch Kondensation der Carboxyl-Gruppen und Hydroxyl-Gruppen der Pektinmoleküle gebildet wird.

2. Die passive, stabile, auf einen Tumor abzielende, krebsbekämpfende Prodrug nach Anspruch 1, **dadurch gekennzeichnet, dass** die Teilchengröße der passiven, stabilen, auf einen Tumor abzielenden, krebsbekämpfenden Prodrug 130 nm bis 180 nm beträgt.

3. Die passive, stabile, auf einen Tumor abzielende, krebsbekämpfende Prodrug nach Anspruch 1 oder 2, **dadurch gekennzeichnet, dass** die Wasserlöslichkeit der passiven, stabilen, auf einen Tumor abzielenden, krebsbekämpfenden Prodrug bei 42 mg/L liegt.

4. Die passive, stabile, auf einen Tumor abzielende, krebsbekämpfende Prodrug nach Anspruch 1, **dadurch gekennzeichnet, dass** die Pektin-Doxorubicin-Kombination durch der Reaktion des kleinmolekulares Pektins mit Doxombicin in der Kombination mit EDC HCl bei 40-60°C und einem pH-Wert von 5-7 erhalten wird.

5. Die passive, stabile, auf einen Tumor abzielende, krebsbekämpfende Prodrug nach Anspruch 4, **dadurch gekennzeichnet, dass** die Molmasse des kleinmolekulares Pektins Pektins bei 10.000 - 30.000 liegt.

6. Die passive, stabile, auf einen Tumor abzielende, krebsbekämpfende Prodrug nach einem von Anspruch 1 bis 5, **dadurch gekennzeichnet, dass** die Pektin-Doxombicin-Kombination in Wasser gegeben, sowie PVP und Glyzerin hinzugefügt wird und all dies gleichmäßig vermengt wird, um die Suspension herzustellen, und Behandlung der Suspension im Ultrahochdruck Nano-Homogenisierer.

7. Die passive, stabile, auf einen Tumor abzielende, krebsbekämpfende Prodrug nach
Anspruch 6, **dadurch gekennzeichnet, dass** die Suspension in dem Ultrahochdruck Nano-Homogenisierer 3 Mal behandelt wird, und zwar zu 120 MPa beim ersten Mal, 180 MPA beim zweiten Mal und 190 MPa beim dritten Mal.

8. Die passive, stabile, auf einen Tumor abzielende, krebsbekämpfende Prodrug nach Anspruch 7, **dadurch gekennzeichnet, dass** die Dosierung des PVP das 1 - 6-fache der Masse der Pektin-Doxombicin-Kombination und die Dosierung des Glyzerins 0,1 - 0,8 % der Masse der Pektin-Doxombicin-Kombination beträgt.

9. Verfahren zur Vorbereitung einer passiven, stabilen, auf einen Tumor abzielenden, krebsbekämpfenden Prodrug, das durch die folgenden Schritte gekennzeichnet ist:
a. Auflösung des gering molekularen Pektins mit einer Molmasse von 5.000 - 45.000 in Wasser, Hinzugabe von Doxombicin-Hydrochlorid, Reaktion mit EDC HCl für 3 - 8 Std. nach gleichmäßiger Vermengung, Dialyse und Trocknung, um eine Pektin-Doxorubicin-Kombination mit einer Molmasse von 100.000 - 1.000.000 zu erhalten; und
b. Hinzugabe der Pektin-Doxombicin-Kombination in Wasser, sowie Hinzugabe von PVP und Glyzerin durch gleichmäßiges vermengen, um eine Suspension herzustellen, sowie diese Suspension in einem Ultrahochdruck Nano-Homogenisierer zu behandeln und um die passive, stabile, auf einen Tumor abzielende, krebsbekämpfende Prodrug mit einer Teilchengröße von 100nm - 200nm zu erhalten.

10. Verfahren zur Herstellung der passiven, stabilen, auf einen Tumor abzielenden, krebsbekämpfenden Prodrug nach Anspruch 9, das **dadurch gekennzeichnet ist, dass** das gering molekulare Pektin durch Auflösung des Pektins in Wasser, Reaktion des Pektins mit einer NaOH-Lösung bei einem ph-Wert von 13, Anpassung des pH-Wert auf ein neutrales Niveau durch konzentrierte Hydrochloridsäure, sowie Abtrennung von Molmasse, erhalten wird.

## Revendications

1. Promédicament anticancéreux passif et solide, ciblant une tumeur, formé par la liaison de pectine et de doxorubicine, **caractérisé par** la réaction entre une molécule de pectine de petite taille ayant un Mw de 5 000 à 45 000 avec de la doxorubicine pour obtenir un conjugué pectine-doxorubicine avec Mw de 100 000 à 1 000 000, préparant le conjugué en une suspension et traitant la suspension dans un nano-homogénéisateur à ultra haute pression pour obtenir le promédicament anticancéreux passif et solide, ciblant une tumeur, ayant une taille de particule de 100 nm à 200 nm et un point de fusion de 220 à 245° C dans lequel la pectine et la doxorubicine sont liées par une liaison amide et la pectine est liée par une liaison ester formée en condensant des groupes carboxyle et des groupes hydroxyle de molécules de pectine.

2. Le promédicament anticancéreux passif et solide, ciblant une tumeur, selon la revendication 1, **caractérisé en ce que** la taille de particule du promédicament anticancéreux passif et solide, ciblant une tumeur est de 130 nm à 180 nm.

3. Le promédicament anticancéreux passif et solide, ciblant une tumeur, selon la revendication 1 ou 2, **caractérisé par le fait que** la solubilité dans l'eau du promédicament anticancéreux passif et solide, ciblant une tumeur est de 42 mg / L.

4. Le promédicament anticancéreux passif et solide, ciblant une tumeur, selon la revendication 1, **caractérisé par le fait que** le conjugué pectine-doxorubicine est obtenu en faisant réagir la petite pectine moléculaire avec de la doxorubicine en présence d'EDC HCl à 40-60° C à pH 5-7.

5. Le promédicament anticancéreux passif et solide, ciblant une tumeur, selon la revendication 4, **caractérisé par le fait que** le Mw de la pectine de petite taille moléculaire est de 10 000 à 30 000.

6. Le promédicament anticancéreux passif et solide, ciblant une tumeur, selon l'une quelconque des revendications 1 à 5, **caractérisé par** l'ajout du conjugué pectine-doxorubicine à de l'eau, l'ajout de PVP et de glycérol, un mélange homogène pour préparer la suspension et le traitement de la suspension dans le nano-homogénéisateur ultra-haute pression.

7. Le promédicament anticancéreux passif et solide, ciblant une tumeur, selon la revendication 6, **caractérisé par le fait que** l'on traite la suspension dans le nano-homogénéisateur ultra haute pression 3 fois, à 120mpa pour la première fois, 180mpa pour la deuxième fois et 190mpa pour la troisième fois.

8. Le promédicament anticancéreux passif et solide, ciblant une tumeur, selon la revendication 7, **caractérisé par le fait que** la posologie du PVP est de 1 à 6 fois la masse du conjugué pectine-doxorubicine et la dose de glycérol est de 0,1 à 0,8 % de la masse du conjugué pectine-doxorubicine.

9. Le procédé de préparation d'un promédicament anticancéreux passif et solide, ciblant une tumeur, **caractérisé par le fait qu'**il comprend les étapes suivantes :
a. Dissolvez une pectine de petite taille moléculaire avec Mw de 5 000 à 45 000 dans de l'eau, ajoutez du chlorhydrate de doxorubicine, faites réagir avec EDC HCl pendant 3 à 8 heures après avoir mélangé de manière uniforme, dialysez et séchez pour obtenir un conjugué pectine-doxorubicine avec Mw de 100 000 à 1 000 000 ; et
b. Ajoutez le conjugué pectine-doxorubicine à de l'eau, ajoutez du PVP et du glycérol, mélangez de manière uniforme pour préparer une suspension et traitez la suspension dans un nano-homogénéisateur ultra haute pression pour obtenir le promédicament anticancéreux passif et solide, ciblant une tumeur ayant une taille de particule de 100 nm à 200 nm.

10. Le procédé de préparation du promédicament anticancéreux passif et solide, ciblant une tumeur, selon la revendication 9, **caractérisé par le fait que** la pectine de petite taille moléculaire est obtenue en dissolvant de la pectine dans l'eau, en faisant réagir la pectine avec une solution de NaOH à pH 13, en ajustant le pH à l'acide chlorhydrique concentré et en ayant une valeur de masse moléculaire de coupure.
